# EUROPEAN PATENT SPECIFICATION

(11) **EP 2 429 462 B1**
(45) Date of publication and mention of the grant of the patent: **20.09.2017**
(21) Application number: 10717423.7
(22) Date of filing: 05.05.2010
(51) Int. Cl.: A61F 2/90

(54) **BIOERODIBLE ENDOPROSTHESIS**
BIOLOGISCH ERODIERBARE ENDOPROTHESE
ENDOPROTHÈSE SENSIBLE À L'ÉROSION BIOLOGIQUE

(30) Priority: 14.05.2009 US 466110
(43) Date of publication of application: 21.03.2012
(73) Proprietor: Boston Scientific Scimed, Inc., Maple Grove, MN 55311-1566 (US)
(72) Inventor: WEBER, Jan, NL-6228 GJ Maastricht (NL); SCHEUERMANN, Torsten, 80803 Munich (DE)
(74) Representative: Peterreins Schley
(86) International application number: PCT/US2010/033648
(87) International publication number: WO 2010/132244

(56) References cited:
- WO-A2-2005/122960
- WO-A2-2008/034007
- US-A1- 2006 106 451
- US-A1- 2007 050 009
- US-A1- 2007 055 362
- US-A1- 2007 254 012

## Description

### TECHNICAL FIELD

This invention relates to endoprosthesis, and more particularly to stents.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism can include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

It is sometimes desirable for an implanted endoprosthesis to erode over time within the passageway. For example, a fully erodible endoprosthesis does not remain as a permanent object in the body, which may help the passageway recover to its natural condition. Erodible endoprostheses can be formed from, e.g., a polymeric material, such as polylactic acid, or from a metallic material such as magnesium, iron or an alloy thereof.

Document US20070254012 discloses an implantble stent with a degradable structural element including an abluminal layer with a faster degradation rate than a luminal layer.

### SUMMARY

The invention features a stent as claimed.

In an aspect, the invention features a stent including a first tubular element formed of a first bioerodible metal composition and a second tubular element formed of a second biodegradable metal composition. The first and second tubular elements are concentrically arranged. The first and second bioerodible metal compositions are different.

In an aspect, the invention features a stent including a first tubular element and a second element arranged to coexpand. The first tubular element is formed of a first bioerodible metal composition, and the second element formed of a second bioerodible metal composition. The first and second bioerodible metal compositions have different erosion rates and a protective coating between at least portions of the two elements to reduce galvanic coupling.

In an aspect, the invention features a stent including a first tubular element formed of a first bioerodible metal composition and second tubular element formed of a second biodegradable metal composition. The first and second tubular elements are concentrically arranged and the first and second bioerodible metal compositions are different.

In an aspect, the invention features a stent including a first tubular element and a second element arranged to coexpand. The first tubular element is formed of first bioerodible metal composition. The second element is formed of a second bioerodible metal composition. The first and second bioerodible metal compositions have different erosion rates and a protective coating between at least portions of the two elements to reduce galvanic coupling.

Embodiments may include one or more of the following as long as it is supported by the claims. The first and second metals are not in direct contact. The first and second metals are separated by a substantially non-conducting material. The non-conducting material is a polymer or a ceramic. The ceramic is selected from an oxide, fluoride, or nitride. The non-conducting material is a coating on the second tubular element, which is arranged radially inward of the first tubular element. The first metal composition has a higher erosion rate than the second metal composition. The first metal composition has higher elastic recoil than the second metal composition. The first metal composition is Mg or a Mg alloy and the second metal composition is iron or an iron alloy. The first tubular member includes a first pattern of wall openings and the second tubular member includes a second pattern of wall openings. The first and second patterns are different. The tubular wall openings of one tubular member are partially occluded by the other tubular member. The thickness of the first and second tubular members is about 125 micron or less. The second tubular member is longer than the first tubular member. The ends of the first tubular member extend beyond the second tubular member. The ends of the first tubular member include a therapeutic agent. The second element is arranged coaxially with the first tubular element. The first element is an arc-shaped element. A plurality of arc-shaped elements are arranged axially along the first tubular member. The arc-shaped element is arranged radially outside the first tubular element.

Embodiments may include one or more of the following advantages. A stent is provided with advantageous mechanical properties, biodegradability, drug delivery characteristics, and/or MRI/fluoroscopic properties. In embodiments, different biodegradable metals are combined in selected arrangements to provide a stent system. For example, a first member of a rapidly eroding but more elastic metal, e.g., Mg is combined with a second metal member of a slowly eroding but stronger and more radiopaque metal, e.g., Fe. The stent initially has sufficient radial strength to maintain lumen patency on deployment in a vessel, then erodes in a controlled manner over a desirable time period. The thickness, pattern, and orientation of the members are selected to provide therapeutic benefit. For example, both members may be thinner than if one or the other metal is used alone. A member formed of a more elastic metal may be supported by a higher strength member to limit elastic recoil. A member formed of the more slowly eroding metal may be thinner so that it is substantially completely eroded within a desired time. The endoprosthesis can have a low thrombogenecity and high initial strength. Lumens implanted with the endoprosthesis can exhibit reduced restenosis.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIGS. 1A-1C are sequential, longitudinal cross-sectional views, illustrating delivery of an endoprosthesis in a collapsed state, expansion of the endoprosthesis, and the deployment of the endoprosthesis in a body lumen.
FIG. 2A is a perspective view of an embodiment of a stent, while FIB. 2B is an exploded view of the stent.
FIG. 3 is a cross-section through a portion of the stent in FIG. 2A.
FIG. 4 is a graph illustrating stent function as a function of time.
FIG. 5 is a perspective view of an embodiment of a stent.

### DETAILED DESCRIPTION

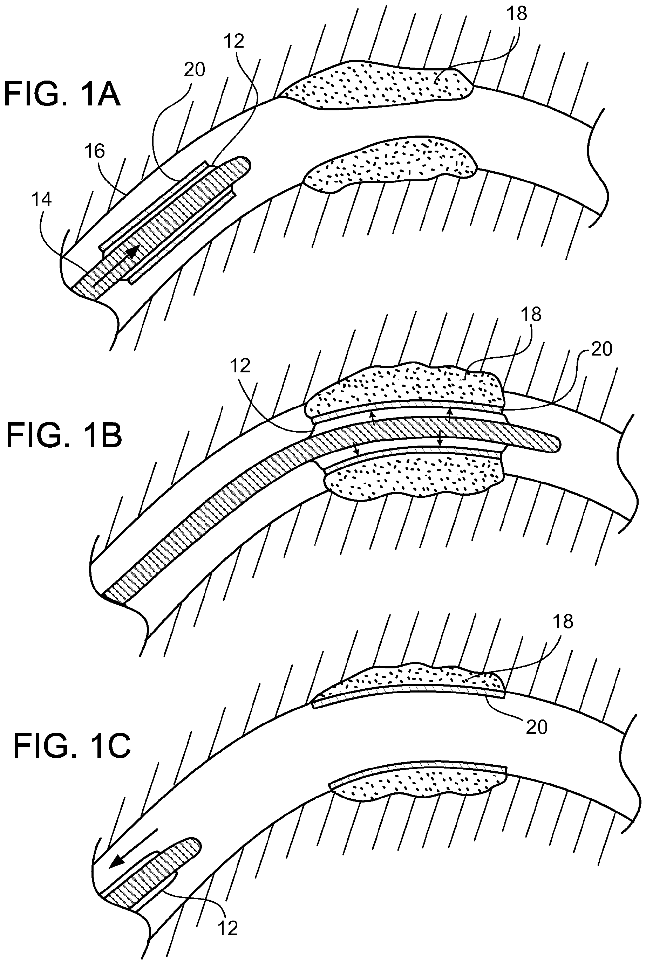

Referring to FIGS. 1A-1C, a stent 20 is placed over a balloon 12 carried near a distal end of a catheter 14, and is directed through the lumen 16 (FIG. 1A) until the portion carrying the balloon and stent reaches the region of an occlusion 18. The stent 20 is then radially expanded, e.g. by inflating the balloon 12, and compressed against the vessel wall with the result that occlusion 18 is compressed, and the vessel wall surrounding it undergoes a radial expansion (FIG. 1B). The pressure is then released from the balloon and the catheter is withdrawn from the vessel (FIG. 1C).

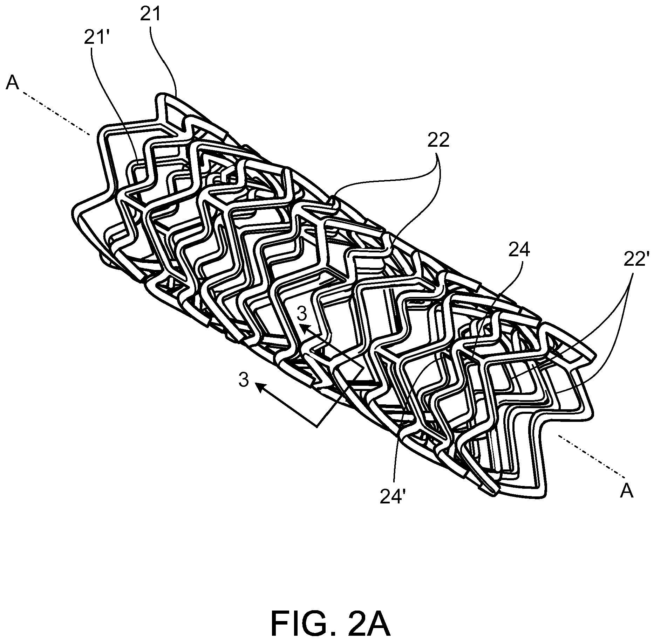

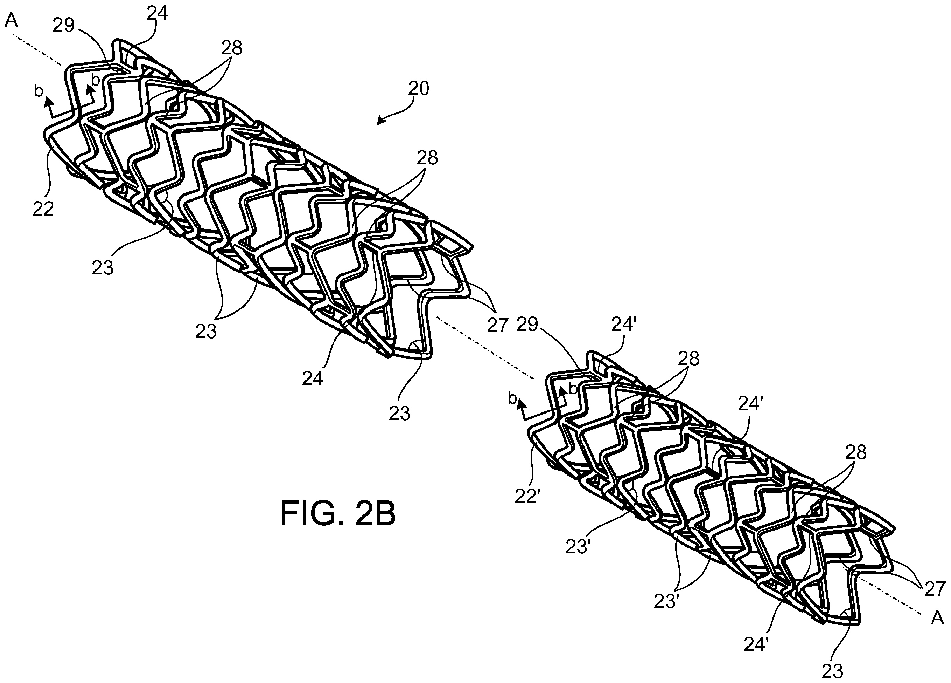

Referring to FIGS. 2A and 2B, an expandable stent 20 can have a tubular stent body arranged around an axis and formed of two coaxially arranged tubular members 21, 21', formed of different bioerodible metal compositions. For example, one member, e.g., the outer member 21, is made of a fast eroding metal, such as Mg, and the other is made of a slower eroding metal, e.g., Fe.

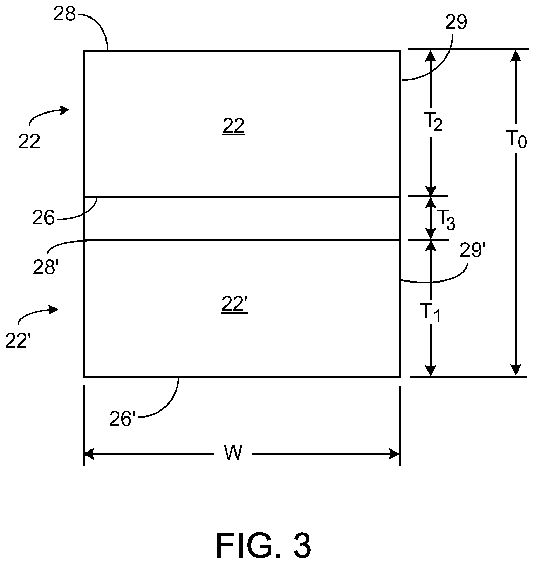

Referring to FIG. 3, a cross section along line 33 in FIG. 2A is provided where tubular members 21, 21' overlap. The member 21' carries an optional coating 27, such as an insulating coating of ceramic or polymer to prevent direct contact between the members 21, 21', and thus reduce galvanic coupling.

Together, the tubular members provide a stent body with an overall thickness, T, and each has a thickness T₁ and T₂, respectively. The coating has a thickness T₃. Each tubular member is defined by a plurality of struts which may be in the form of bands 22, 22' and a plurality of connectors 24, 24' that extend between and connect adjacent bands. During use, bands 22, 22' can be expanded from an initial, smaller diameter to a larger diameter to contact stent 20 against a wall of a vessel, thereby maintaining the patency of the vessel. Connectors 24, 24' can provide stent 20 with flexibility and conformability that allow the stent to adapt to the contours of the vessel. Stent body 20, bands 22 and connectors 24 can have a luminal surface 26, an abluminal surface 28, and a sidewall surface 29. In embodiments, the bands and/or connectors, have a width, W. The coating 27 can in addition or in the alternative be on the member 22', and can cover as well one or more of the other surfaces, side wall or abluminal and/or luminal.

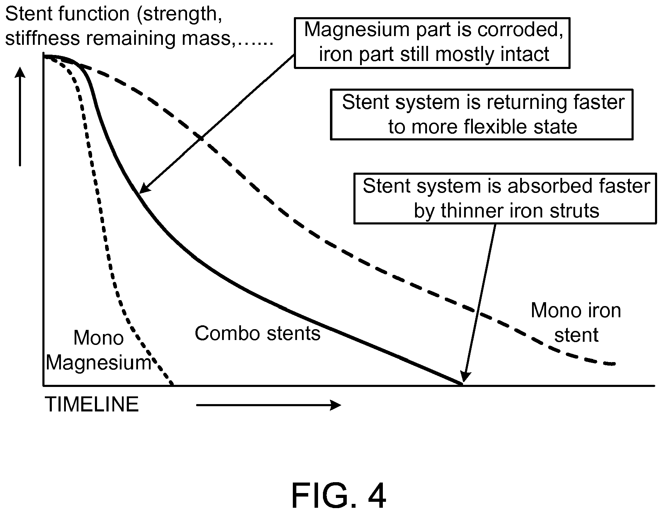

Referring to FIG. 4, the composition, dimensions, and design of the members 21, 21' are selected to enhance the performance of the stent. The graph compares the performance of a stent with a combination of two tubular members, one each formed of Mg and Fe, with a single, monostent tubular member made of either metal. The properties of each metal can be utilized in combination to provide a stent with advantageous properties. Magnesium may corrode too fast, iron too slow. Magnesium furthermore is not as radiopaque and has a larger recoil. Using two tubular members, both members can be made thinner and less dense in cell pattern than normally would be the case for a monostent. Iron is stronger than magnesium, so for example, the strut size T₂, of magnesium can be reduced, i.e. from 150 micrometer to less than 100. Part of the initial radial forces is supplied by the magnesium stent, which allows the iron member pattern to be less dense than a monostent and use this design advantage to make thinner iron struts. Thinner struts will corrode faster addressing a disadvantage of iron. By using a less strong iron member, the stented vessel returns faster to its natural flexibility. The recoil of Magnesium can be mitigated by placing the magnesium member on the outside of the iron. The thickness of the iron, struts T₁ may be, for example, about 50 micron, e.g., 30 micron or less and/or 5 to 10 micron or more. The radiopacity of magnesium is relatively low, but radiopacity is enhanced by the iron. Similarly, with less iron than a monostent, MRI visibility will be enhanced. In addition, although both elements (magnesium and iron) are systemically non-toxic considering the implanted mass, both elements have separate biological removal pathways, so using them in combination reduces the total amount of each element. A drug coating can be provided, e.g., on the faster eroding magnesium, to make the magnesium last longer than the iron inner stent. The drug coating can be located only at the proximal and distal ends of the stent to provide drug release distally and proximally in the vessel. Because a direct contact between magnesium and iron would lead to a galvanic couple, a protective coating is provided between the two metals. For example providing the magnesium member can include a MgF coating, and this would serve a dual function by delaying the onset of corrosion in the magnesium stent. Other protective coatings include polymers or ceramics, e.g., metal oxides or nitrites. The tubular members can be coextensive or partially coextensive. For example, a first tubular member may be longer than the other such that the first extends beyond the second both distally and proximally. The first and second tubular member may be along the axis, such that the first extends beyond the second proximally, and the second extends beyond the first distally.

### Other Embodiments:

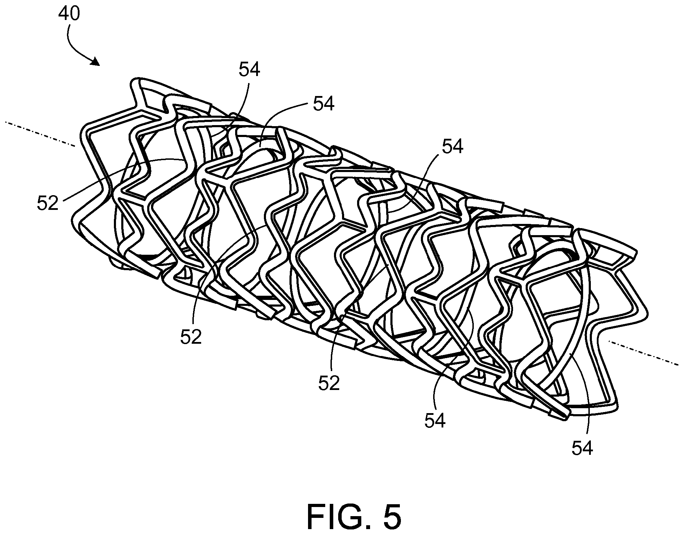

Referring to FIG. 5, in another embodiment, a stent 40 includes a tubular member 52 and a series of separate arc-shaped elements 54 such as rings or partial rings, along the tubular body. The member 52 and element 54 are formed of different bioerodible metal compositions. The elements 54 can be friction fit or fixed to the tubular body, e.g., using a polymer, adhesive, or the like. The elements can be discrete as shown, or connected. The elements can be outside or inside the tubular body, or both. The elements can be spaced to, e.g., facilitate treatment near a bifurcation. For example, the portion of stent adjacent a bifurcation may be free of elements, which facilitates blood flow to the bifurcation and encourages more rapid erosion, which further enhances flow.

The tubular members are preferably formed as discrete elements that can be held together by friction or fixed by a material that is biostable or bioerodible, e.g., a metal or a polymer, or fixed by a mechanical interlocking arrangement. For example, fingers or tabs can be formed on one or both of the members, e.g. by laser cutting, that are bent around the other member to keep the two members together. One or both of the members can be of tapered wall thickness. For example, one tubular member may have a wall thickness that tapers from 40 to 80 microns proximal to distal and the other member tapered 80 to 40 microns proximal to distal. The tapers can be made by grinding a tube and then forming it into a stent.

The members can be deployed in a lumen simultaneously, as described above, or sequentially. In sequential deployment, a first member is expanded at a treatment site and then the second member is expanded inside the first member. The members can be delivered on separate balloon catheters or a single catheter that includes two balloons along its length. After the first balloon is expanded to expand the first tubular member, the catheter is advanced (or retracted) to bring the second tubular member inside the first, and the second tubular member is expanded to expand the second tubular member into engagement with the first.

A stent is bioerodible if the stent or a portion thereof exhibits substantial mass or density reduction or chemical transformation, after it is introduced into a patient, e.g., a human patient. Mass reduction can occur by, e.g., dissolution of the material that forms the stent and/or fragmenting of the stent. Chemical transformation can include oxidation/reduction, hydrolysis, substitution, and/or addition reactions, or other chemical reactions of the material from which the stent or a portion thereof is made. The erosion can be the result of a chemical and/or biological interaction of the stent with the body environment, e.g., the body itself or body fluids, into which it is implanted. The erosion can also be triggered by applying a triggering influence, such as a chemical reactant or energy to the stent, e.g., to increase a reaction rate. For example, a stent or a portion thereof can be formed from an active metal, e.g., Mg or Fe or an alloy thereof, and which can erode by reaction with water, producing the corresponding metal oxide and hydrogen gas; a stent or a portion thereof can also be formed from a bioerodible polymer, or a blend of bioerodible polymers which can erode by hydrolysis with water. Fragmentation of a stent occurs as, e.g., some regions of the stent erode more rapidly than other regions. The faster eroding regions become weakened by more quickly eroding through the body of the endoprosthesis and fragment from the slower eroding regions.

Preferably, the erosion occurs to a desirable extent in a time frame that can provide a therapeutic benefit. For example, the stent may exhibit substantial mass reduction after a period of time when a function of the stent, such as support of the lumen wall or drug delivery, is no longer needed or desirable. In certain applications, stents exhibit a mass reduction of about 10 percent or more, e.g. about 50 percent or more, after a period of implantation of about one day or more, about 60 days or more, about 180 days or more, about 600 days or more, or about 1000 days or less. Erosion rates can be adjusted to allow a stent to erode in a desired sequence by either reducing or increasing erosion rates. For example, regions can be treated to increase erosion rates by enhancing their chemical reactivity, e.g., coating portions of the stent with a silver coating to create a galvanic couple with the exposed, uncoated Iron surfaces on other parts of the stent. Alternatively, regions can be treated to reduce erosion rates, e.g., by using coatings.

A coating can be deposited or applied over the surface of stent to provide a desired function. Examples of such coatings include a tie layer, a biocompatible outer coating, a radiopaque metal or alloy, and/or a drug-eluting layer. A stent can be incorporated with at least one releasable therapeutic agent, drug, or pharmaceutically active compound to inhibit restenosis, such as paclitaxel, or to treat and/or inhibit pain, encrustation of the stent or sclerosing or necrosing of a treated lumen. The therapeutic agent can be a genetic therapeutic agent, a non-genetic therapeutic agent, or cells. The therapeutic agent can also be nonionic, or anionic and/or cationic in nature. Examples of suitable therapeutic agents, drugs, or pharmaceutically active compounds include anti-thrombogenic agents, antioxidants, anti-inflammatory agents, anesthetic agents, anticoagulants, and antibiotics, as described in U.S. Patent No. 5,674,242; U.S. Patent Publication No. 2003-0003220; U.S. Patent Publication No. 2005-0251249; and U.S. Patent Publication No. 2003-0185895. Representative approaches disperse the therapeutic agent, drug, or a pharmaceutically active compound in a polymeric coating carried by a stent. The therapeutic agent, drug, or a pharmaceutically active compound can be directly incorporated into the pores generated by plasma immersion ion implantation treatment on the surface of a stent, thereby eliminating the use of extra coatings.

In some embodiments, the stent can include one or more bioerodible metals, such as magnesium, zinc, iron, or alloys thereof. The stent can include bioerodible and non-bioerodible materials. The stent can have a surface including bioerodible metals, polymeric materials, or ceramics. The stent can have a surface including an oxide of a bioerodible metal. Examples of bioerodible alloys also include magnesium alloys having, by weight, 50-98% magnesium, 0-40% lithium, 0-1% iron and less than 5% other metals or rare earths; or 79-97% magnesium, 2-5% aluminum, 0-12% lithium and 1-4% rare earths (such as cerium, lanthanum, neodymium and/or praseodymium); or 85-91% magnesium, 6-12% lithium, 2% aluminum and 1% rare earths; or 86-97% magnesium, 0-8% lithium, 2-4% aluminum and 1-2% rare earths; or 8.5-9.5% aluminum, 0.15%-0.4% manganese, 0.45-0.9% zinc and the remainder magnesium; or 4.5-5.3% aluminum, 0.28%-0.5% manganese and the remainder magnesium; or 55-65% magnesium, 30-40% lithium and 0-5% other metals and/or rare earths. Bioerodible magnesium alloys are also available under the names AZ91D, AM50A, and AE42. Other bioerodible alloys are described in Bolz, U.S. Patent No. 6,287,332 (e.g., zinc-titanium alloy and sodium-magnesium alloys); Heublein, U.S. Patent Publication No. 2002-0000406; and Park, Science and Technology of Advanced Materials, 2, 73-78 (2001). In particular, Park describes Mg-X-Ca alloys, e.g., Mg-Al-Si-Ca, Mg-Zn-Ca alloys. Examples of bioerodible polymers include polydioxanone, polycaprolactone, polygluconate, polylactic acid-polyethylene oxide copolymers, modified cellulose, collagen, poly(hydroxybutyrate), polyanhydride, polyphosphoester, poly(amino acids), poly-L-lactide, poly-D-lactide, polyglycolide, poly(alpha-hydroxy acid), and combinations thereof.

A stent can also include non-bioerodible materials. A reason to combine a bioerodible with a non-bioerodible is that after erosion of the bioerodible material, left in the body is the non-bioerodible member which can be more flexible than an ordinary one-piece stent out of, e.g., stainless steel. This also allows the use of some stent materials like Titanium or tantalum alloys which might be less suitable to build an entire tent out of from a mechanical perspective.

Examples of suitable non-bioerodible materials include stainless steels, platinum enhanced stainless steels, titanium, tantalum, cobalt-chromium alloys, nickel-titanium alloys, noble metals and combinations thereof. In some embodiments, stent 20 can include bioerodible and non-bioerodible portions. In some embodiments, non-bioerodible or biostable metals can be used to enhance the X-ray visibility of bioerodible stents. The bioerodible stent main structure of a stent can be combined with one or more biostable marker sections. The biostable marker sections can include, for example, Gold, Platinum or other high atomic weight elements. The biostable marker sections can provide enhance visibility and radiopacity and can provide a structural purpose as well. Coatings such as ceramics, metals and polymers can be formed by deposition techniques such as PVD, solgel, and the like. Suitable techniques are described in U.S. Patent Publication No. 2008-0294236; U.S. Patent Publication No. 2008-0294246; U.S. Patent Publication No. 2010-0057188; and U.S. Patent Publication No. 2009-0319032.

A stent can have any desired shape and size (e.g., superficial femoral artery stents, coronary stents, aortic stents, peripheral vascular stents, gastrointestinal stents, urology stents, and neurology stents). Depending on the application, a stent can have an expanded diameter of about 1 mm to about 46 mm. For example, a coronary stent can have an expanded diameter of about 2 mm to about 6 mm; a peripheral stent can have an expanded diameter of about 5 mm to about 24 mm; a gastrointestinal and/or urology stent can have an expanded diameter of about 6 mm to about 30 mm; a neurology stent can have an expanded diameter of about 1 mm to about 12 mm; and an abdominal aortic aneurysm stent and a thoracic aortic aneurysm stent can have an expanded diameter of about 20 mm to about 46 mm. The stent can be self-expandable, balloon-expandable, or a combination of self-expandable and balloon-expandable (e.g., as described in U.S. Patent No. 5,366,504). The stent can have any suitable transverse cross-section, including circular and non-circular (e.g., polygonal such as square, hexagonal or octagonal). An overlapped stent arrangement is described in U.S. Patent Publication No. 2005-0278017.

A stent can be implemented using a catheter delivery system. Catheter systems are described in, for example, Wang U.S. Patent No. 5,195,969; Hamlin U.S. Patent No. 5,270,086; and Raeder-Devens, U.S. Patent No. 6,726,712. Commercial examples of stents and stent delivery systems include Radius^{®}, Symbiot^{®} or Sentinol^{®} system, available from Boston Scientific Scimed, Maple Grove, MN.

A stent can be a part of a covered stent or a stent-graft. For example, a stent can include and/or be attached to a biocompatible, non-porous or semi-porous polymer matrix made of polytetrafluoroethylene (PTFE), expanded PTFE, polyethylene, urethane, or polypropylene. In addition to vascular lumens, a stent can be configured for non-vascular lumens. For example, it can be configured for use in the esophagus or the prostate. Other lumens include biliary lumens, hepatic lumens, pancreatic lumens, uretheral lumens and ureteral lumens.

## Claims

1. A stent, comprising
a first tubular element (21) formed of a first bioerodible metal composition and second tubular element (21') formed of a second bioerodible metal composition, the first and second tubular elements (21, 21') are concentrically arranged;
wherein the first and second bioerodible metal compositions are different and **characterised in that** the first metal composition is Mg or a Mg alloy and the second metal composition is iron or an iron alloy; and
wherein a protective coating is provided between the two metals to reduce galvanic coupling.

2. The stent of claim 1 wherein the first and second metals are not in direct contact.

3. The stent of claim 1 wherein the first and second metals are separated by a substantially non-conducting material.

4. The stent of claim 3 wherein the non-conducting material is a polymer or a ceramic, the ceramic selected from an oxide, fluoride, or nitride.

5. The stent of claim 2 wherein the non-conducting material is a coating on the second tubular element, which is arranged radially inward of the first tubular element.

6. The stent of claim 1 wherein the first metal composition has a higher erosion rate than the second metal composition.

7. The stent of claim 1 wherein the first metal composition has higher elastic recoil than the second metal composition.

8. The stent of claim 1 wherein the first tubular member (21) includes a first pattern of wall openings and the second tubular member (21') includes a second pattern of wall openings.

9. The stent of claim 8 wherein the first and second patterns are different.

10. The stent of claim 8 wherein the tubular wall openings of one tubular member are partially occluded by the other tubular member.

11. The stent of claim 1 wherein the thickness of the first and second tubular members (21, 21') is about 125 micron or less.

12. The stent of claim 1 wherein the first tubular member (21) is longer than the second tubular member (21').

13. The stent of claim 12 wherein the ends of the first tubular member (21) extend beyond the second tubular member (21').

14. The stent of claim 13 wherein the ends of the first tubular member (21) include a therapeutic agent.

## Patentansprüche

1. Stent, umfassend
ein erstes rohrförmiges Element (21), gebildet aus einer ersten bioerodierbaren Metallzusammensetzung, und ein zweites rohrförmiges Element (21'), gebildet aus einer zweiten bioerodierbaren Metallzusammensetzung, wobei das erste und das zweite rohrförmige Element (21,21') konzentrisch angeordnet sind;
wobei die erste und die zweite bioerodierbare Metallzusammensetzung verschieden sind und **dadurch gekennzeichnet sind, dass** die erste Metallzusammensetzung Mg oder eine Mg-Legierung ist und die zweite Metallzusammensetzung Eisen oder eine Eisenlegierung ist; und
wobei eine Schutzbeschichtung zwischen den beiden Metallen bereitgestellt ist, um galvanische Kopplung zu verringern.

2. Stent gemäß Anspruch 1, wobei das erste und das zweite Metall nicht in direktem Kontakt stehen.

3. Stent gemäß Anspruch 1, wobei das erste und das zweite Metall durch ein im Wesentlichen nichtleitendes Material getrennt sind.

4. Stent gemäß Anspruch 3, wobei das nichtleitende Material ein Polymer oder eine Keramik ist, wobei die Keramik ausgewählt ist aus einem Oxid, Fluorid oder Nitrid.

5. Stent gemäß Anspruch 2, wobei das nichtleitende Material eine Beschichtung auf dem zweiten rohrförmigen Element ist, die radial innen bezogen auf das erste rohrförmige Element angeordnet ist.

6. Stent gemäß Anspruch 1, wobei die erste Metallzusammensetzung eine höhere Erosionsrate als die zweite Metallzusammensetzung aufweist.

7. Stent gemäß Anspruch 1, wobei die erste Metallzusammensetzung eine höhere elastische Rückstellung als die zweite Metallzusammensetzung aufweist.

8. Stent gemäß Anspruch 1, wobei das erste rohrförmige Element (21) eine erste Struktur von Wandöffnungen aufweist und das zweite rohrförmige Element (21') eine zweite Struktur von Wandöffnungen aufweist.

9. Stent gemäß Anspruch 8, wobei die erste und die zweite Struktur verschieden sind.

10. Stent gemäß Anspruch 8, wobei die Öffnungen der rohrförmigen Wand eines rohrförmigen Elements von dem anderen rohrförmigen Element teilweise verdeckt sind.

11. Stent gemäß Anspruch 1, wobei die Dicke des ersten und des zweiten rohrförmigen Elements (21, 21') etwa 125 Mikrometer oder weniger beträgt.

12. Stent gemäß Anspruch 1, wobei das erste rohrförmige Element (21) länger als das zweite rohrförmige Element (21') ist.

13. Stent gemäß Anspruch 12, wobei die Enden des ersten rohrförmigen Elements (21) über das zweite rohrförmige Element (21') hinausragen.

14. Stent gemäß Anspruch 13, wobei die Enden des ersten rohrförmigen Elements (21) ein therapeutisches Mittel enthalten.

## Revendications

1. Endoprothèse, comprenant :
un premier élément tubulaire (21) formé d'une première composition de métal bioérodable et un second élément tubulaire (21') formé d'une seconde composition de métal bioérodable, les premier et second éléments tubulaires (21,21') étant disposés de façon concentrique ;
dans laquelle les première et seconde compositions de métal bioérodables sont différentes et **caractérisées en ce que**
la première composition de métal est du magnésium (Mg) ou un alliage de magnésium et la seconde composition de métal est du fer ou un alliage de fer ; et
dans laquelle un revêtement de protection est disposé entre les deux métaux pour réduire un couplage galvanique.

2. Endoprothèse selon la revendication 1, dans laquelle les premier et second métaux ne sont pas en contact direct.

3. Endoprothèse selon la revendication 1, dans laquelle les premier et second métaux sont séparés par un matériau sensiblement non conducteur.

4. Endoprothèse selon la revendication 3, dans laquelle le matériau non conducteur est un polymère ou une céramique, la céramique étant sélectionnée parmi un oxyde, un fluorure ou un nitrure.

5. Endoprothèse selon la revendication 2, dans laquelle le matériau non conducteur est un revêtement sur le second élément tubulaire qui est disposé radialement vers l'intérieur du premier élément tubulaire.

6. Endoprothèse selon la revendication 1, dans laquelle la première composition de métal présente une vitesse d'érosion plus élevée que celle de la seconde composition de métal.

7. Endoprothèse selon la revendication 1, dans laquelle la première composition de métal présente un recul élastique plus important que celui de la seconde composition de métal.

8. Endoprothèse selon la revendication 1, dans laquelle le premier élément tubulaire (21) comprend un premier motif d'ouvertures de paroi et le second élément tubulaire (21') comprend un second motif d'ouvertures de paroi.

9. Endoprothèse selon la revendication 8, dans laquelle les premier et second motifs sont différents.

10. Endoprothèse selon la revendication 8, dans laquelle les ouvertures de paroi tubulaires d'un élément tubulaire sont partiellement occluses par l'autre élément tubulaire.

11. Endoprothèse selon la revendication 1, dans laquelle l'épaisseur des premier et second éléments tubulaires (21, 21') est égale ou inférieure à environ 125 microns.

12. Endoprothèse selon la revendication 1, dans laquelle le premier élément tubulaire (21) est plus long que le second élément tubulaire (21').

13. Endoprothèse selon la revendication 12, dans laquelle les extrémités du premier élément tubulaire (21) s'étendent au-delà du second élément tubulaire (21').

14. Endoprothèse selon la revendication 13, dans laquelle les extrémités du premier élément tubulaire (21) comprennent un agent thérapeutique.
